# EUROPEAN PATENT SPECIFICATION

(11) **EP 4 142 855 B1**
(45) Date of publication and mention of the grant of the patent: **29.10.2025**
(21) Application number: 21726760.8
(22) Date of filing: 28.04.2021
(51) Int. Cl.: A61B 5/00, A61M 27/00, A61B 5/03

(54) **A METHOD AND SYSTEM TO CONTROL A HYDROCEPHALUS SHUNT SYSTEM**
VERFAHREN UND SYSTEM ZUR STEUERUNG EINES HYDROCEPHALUS-NEBENSCHLUSSSYSTEMS
PROCÉDÉ ET SYSTÈME POUR COMMANDER UN SYSTÈME DE DÉRIVATION D'HYDROCÉPHALIE

(30) Priority: 29.04.2020 US 202016862090
(43) Date of publication of application: 08.03.2023
(73) Proprietor: Medtronic Xomed, Inc., Jacksonville, FL 32216 (US)
(72) Inventor: BERTRAND, Jeff William, Goleta, California 93117 (US)
(74) Representative: Viering, Jentschura & Partner mbB Patent- und Rechtsanwälte
(86) International application number: PCT/US2021/029661
(87) International publication number: WO 2021/222417

(56) References cited:
- WO-A1-02/07596
- CN-A- 108 310 477
- US-A1- 2015 094 644

## Description

### FIELD

Disclosed is a system and method for controlling a shunt system, in particularly directed to an automatic system and method for controlling a hydrocephalus shunt.

### BACKGROUND

This section provides background information related to the present disclosure which is not necessarily prior art.

A subject, such as a human subject, may be treated for various conditions. The conditions may include an overproduction of a certain material within a patient and/or an inability to clear or drain a selected material. In certain subjects, for example, an obstruction to outflow, an insufficient resorption, and/or an overproduction of cerebral spinal fluid (CSF) may lead to a condition referred to as hydrocephalus. Hydrocephalus can often be treated with a shunt system configured to allow a drainage of CSF from ventricles within a brain to a remote or different portion of the subject.

Exemplary systems are disclosed in WO 02/07596 A1, CN 108 310 477 A, and US 2015/094644 A1.

### SUMMARY

According to the present invention, a system for controlling a hydrocephalus shunt system according to claim 1 is provided. Further embodiments are defined in the dependent claims.

This section provides a general summary of the disclosure, and is not a comprehensive disclosure of its full scope or all of its features.

A subject may be treated for hydrocephalus with a shunt system. In a shunt system an inlet catheter may be position in or at a ventricle of a brain. The inlet catheter includes passages that may allow a fluid to flow into the inlet catheter from a ventricle. The fluid may then pass through the inlet catheter and to and through an outlet catheter to drain to a selected portion of the subject. In various embodiments, the outlet catheter may be positioned within a peritoneal area of the subject or a vascular area of the subject. Generally, the outlet or drainage catheter is positioned in an area with high blood flow supply.

The shunt system allows for flow of cerebral spinal fluid (CSF) from a source, such as a ventricle, through the inlet catheter and the outlet catheter. The shunt system, therefore, allows for a flow of the CSF to maintain or achieve a selected volume or pressure within the brain and/or a selected or optimal outcome or result for the subject. Optimal or selected outcomes may include lack or reduction of pain, lack of nausea, increase or normal cognitive function, etc.

Disclosed is a system that allows for controlling the flow of the CSF through the shunt system based upon a feedback. The shunt system may include a portion or system that may be adjusted over time to achieve different or varying flows. The shunt system may, for example, include a valve or pump. Various sensors may sense activity within the subject, such as the brain, to alter the controlled portion of the shunt system to achieve different flows based upon selected inputs.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
Fig. 1 is a schematic illustration of a subject with a shunt system;
Fig. 2 is a schematic diagram of a control system for a shunt system, according to various embodiments;
Fig. 3 is a flow chart regarding an operation of the shunt system by the control system, according to various embodiments; and
Fig. 4 is a flow chart of operation of a shunt system including clinician input, according to various embodiments.

Corresponding reference numerals indicate corresponding parts throughout the several views of the drawings.

### DETAILED DESCRIPTION

Example embodiments will now be described more fully with reference to the accompanying drawings.

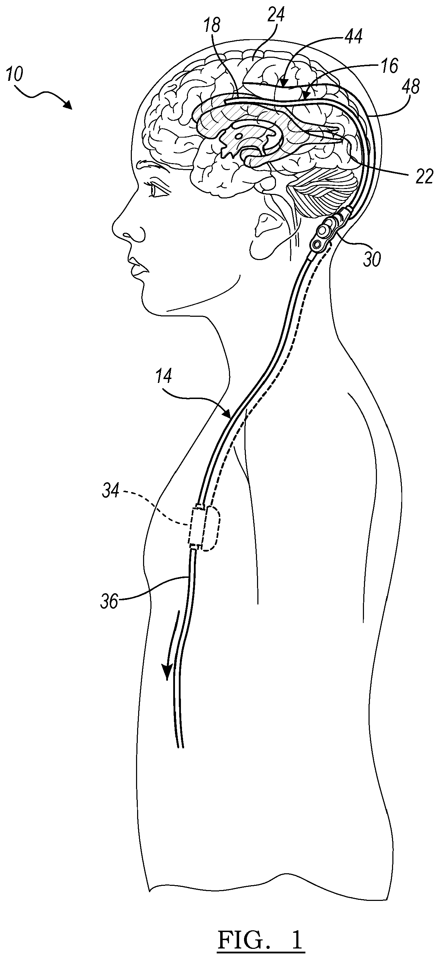

With initial reference to Fig. 1, a subject 10 may have a shunt assembly or system 14 implanted or positioned within the subject 10. The shunt assembly 14 may include an inlet catheter 16 that has an inlet area or portion 18 that is positioned within a ventricle 22. The ventricle 22 may be a part of a brain 24 of the subject 10. The ventricle 22 may generally hold and/or produce cerebral spinal fluid (CSF). The CSF may generally drain or reabsorb into a subject. The subject 10, however, may overproduce CSF and/or have a blockage of f low of CSF from within the brain 24, such as within the ventricle 22, to an area away from the brain 24. Accordingly, the shunt system 14 may be implanted in the subject 10 to assist in the drainage of the CFS from the ventricle 22 to a different location.

Accordingly, the inlet catheter 16 having the inlet end 18 within the ventricle 22 may extend to a flow control assembly. The flow control assembly may include a valve 30 and/or a pump assembly 34. It is understood that the valve 30 or the pump assembly 34 may be implanted in different subjects separately, rather than together, further listed here for clarity of the current discussion. It is further understood, however, that both the valve 30 and the pump 34 may be implanted in a single subject.

The outlet catheter 16 may connect with the valve 30 and/or the pump 34 to allow the CSF to drain through the outlet catheter 14, generally in the direction of arrow 38 to a selected portion of subject 10, such as the peritoneal cavity. When the valve 30 is installed the valve 30 may select a flow based upon a flow rate and/or pressure experienced at the valve 30. Generally, the valve 30 may be selected to open at a selected pressure to allow the CSF to flow from the ventricle to the outlet catheter 38. The pump 34 may also select a flow rate and/or pressure and may actively move fluid through the shunt system 14 through the outlet catheter 36.

In various embodiments, therefore, the shunt system 14 allows fluid to flow from the ventricle 22 to a selected outlet location, such as the peritoneal cavity. The operation of the valve 30 and/or the pump 34 may be based upon a predetermined selected flow rate and/or pressure. In various embodiments, a sensor assembly may include a sensor lead 44 that may be positioned in the brain 24. The sensor lead 44 positioned within the brain 24 may sense various features, such as brain activity within the brain 24. The sensor 44 may be used to generate a sensor signal that is used as feedback to change the flow rate with either or both of the valve 30 and/or the pump 34.

The sensor lead 44 may connect (e.g. send a signal for operation of) to the valve 30 and/or the pump 34. In various embodiments, the sensor lead 44 may be at least partially incorporated into the inlet catheter 16 and/or the outlet catheter 36 to allow for connection to the valve 30 and/or the pump 34. Accordingly, the sensor lead 44 may be positioned within the brain 24 to sense brain activity, also referred to as physiological activity, of the brain 24.

Briefly, the sensor 44 may be used to sense a selected brain activity and/or function. The sensor 44 may be used to sense or record the selected brain function or activity. For example, the sensor 44 may include one or more electrodes to sense or receive electrical activity from the brain 24. The sensor 44 may then generate or send a sensor signal that may be a signal that may be sent from the sensor and/or provided from the sensor along a lead or connector 48 to the selected one of the valve 30 and/or the pump 34. As discussed further herein, the sensor signal may be used to control, such as maintain or alter the valve 30 and/or the pump 34. The sensor signal may be used to, for example, alter or change an operation of the valve 30 and/or the pump 34 based upon the sensor signal. Thus, the valve 30 and/or the pump 34 may be operated in an active manner based upon feedback including the sensor signal from the sensor 44.

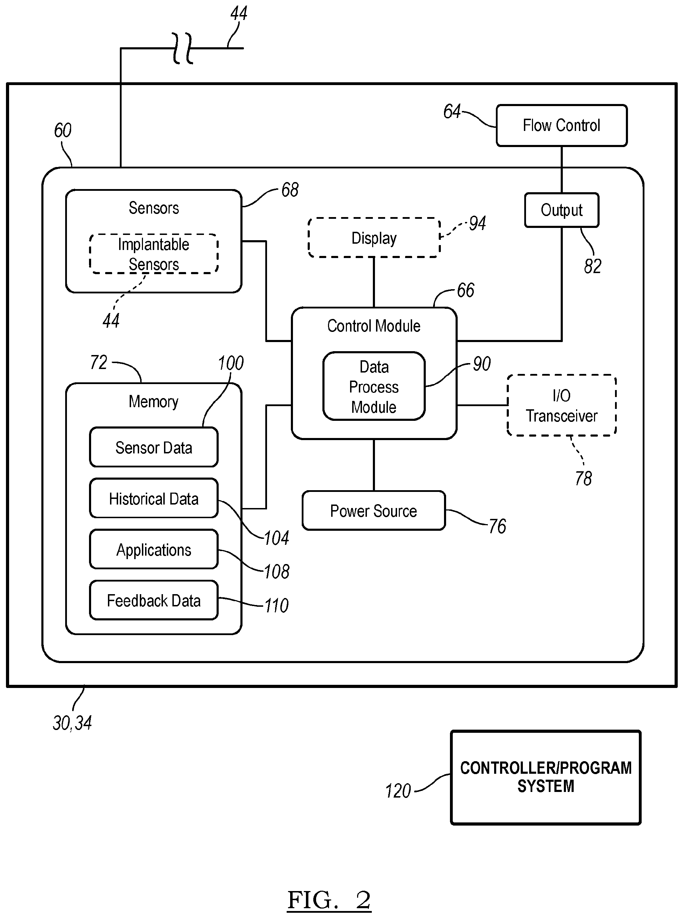

With continuing reference to Fig. 1, and additional reference to Fig. 2, a body control device, also referred to as a control system, 60 is illustrated. The control system 60 may be incorporated into either or both of the valve 30 and/or the pump 34. For the present discussion, reference will be made to the valve 30, but it is understood that the control system 60 may be incorporated into either or both of the valve 30 and/or or the pump 34. Similarly, the present disclosure will refer to the valve 30 however, it is understood, that the shunt system 40 may include either or both of the valve 30 and/or the pump 34, unless specifically stated otherwise.

The control system 60 may be incorporated into the valve 30. The valve system 30 may include a flow control portion or assembly 64 that may be included or incorporated into the valve 30. In various embodiments, the valve 30, for example, may include a ball portion that engages a valve seat to control flow through the valve 30. The ball member may be held in place with a selected resilient portion, such as a spring. Force applied to the spring may increase or decrease the spring force or force applied by the spring to the ball. The force applied to the ball onto the valve seat may control or select the opening pressure of the flow control 64. Accordingly, adjustment of the flow control 64 may adjust a pressure needed to open the valve 30 to allow flow of the CSF from the ventricle 22 through the valve 30 of the shunt system 14.

In various embodiments, the control system 60 may include a feedback, such as a closed feedback loop, to control the flow control 64 of the valve 30. Again, it is understood, that the control system 60 may also control the flow control 64 in the pump 34. In various embodiments, the flow control 64 of the pump 34 may include a pump power, duty cycle, speed, volume or flow rate, or the like.

The control system 60 may include various components, such as a control module 66, one or more sensors or sensor connections or inputs 68, a memory system or module 72, a power source 76. Further, the control module may include one or more outputs, such as a wireless output transceiver 78 and/or a wired output 82. In various embodiments, for example, the output 82 may allow for output from the control system 60 to a flow control 64. The output 82 may include a wired or hard connection (e.g. a trace) in an integrated circuit board (ICB) or other appropriate wired connection. It is understood, however, that the transceiver 78 is optional and may also communicate with the flow control 64 in an appropriate manner. Accordingly, the control system 60 may include one or more outputs for sending instructions or signals and/or receiving instructions or signals.

The control module 66 may include a data processing module 90 that may analyze and/or evaluate data collected with the sensor 68 and/or data received via the transceiver which may also be an input system 78. As discussed above, the sensor 68, including the implanted sensor 44, and the control system 60 may include an input from the implanted sensor 44 that may be positioned in the brain 24. The sensors 68, therefore, may provide inputs to the control module 66 for analysis and/or evaluation, as discussed further herein.

The analysis and/or evaluation of data by the data process module 90 may be performed by and/or augmented by data and/or instructions stored in the memory 72. The memory 72 may be internal to the control system 60, as illustrated in Fig. 2, and/or may be external to the control system 60. For example, the memory 72 may be accessed with the transceiver 78.

The control module 66 may further include a display or further output 94. The display 94, if included, may provide output regarding the selected setting, pressure, historical or sensed data, or the like. It is understood, however, that the display 94 is not required and may not be included.

The memory 72 may include a system or mechanism to store a selected data, such as a sensor data 100 from the sensor 44. The sensor data 100 may be immediate data, such as current data used to set the current flow control 64, or any appropriate sensor data. Further, historical data 104 may also be saved on the memory 72. Historical data may include a selection of data regarding the historical over a set time period of sensor data and/or flow control settings. The historical data 104 may be output from the control system 60 for analysis, such as for further treatment, settings or controls of the control system 60 for the flow control 64, or other appropriate purposes.

The memory 72 may further include applications 108 that may be executed by the control module 66 for various purposes, such as for controlling the flow control 64. Applications may include various features, such as those discussed further herein, such as for collecting data from the sensors 68, analyzing or processing the sensor data, and/or controlling the flow control 64 based upon selected instructions and/or input from the sensors 44.

Further, the memory may include a feedback data 110 that may include data regarding feedback for control of the flow control 64 and/or a change in sensor inputs or sensed information by the sensors 44. Also, the applications 108 may be executed to analyze the feedback data 110 to assist in selecting control or output for the flow control 64.

The transceiver 78 may include a physical layer that is configured to transmit and/or receive signals from the control module 66 and/or an external control/program module or system 120. The control/program system 120 may be operated by a selected user, such as a surgeon or clinician, to assist in operation of the shunt system 14. For example, the control/program system 120 may be used to generate and/or update the applications 108, provide applications 108 including new applications, receive sensor data, historical data, and/or feedback data 100, 104, 110 for analysis and/or treatment of the subject 10, or other appropriate features. The control/program system 120 may assist in augmenting operation of the control system 60 of the valve 30 to achieve a selected or optimal operation of the shunt system 14.

Accordingly, the shunt system 14 may be implanted in the subject 10 at a selected time and operated for a time period based upon the control module 66 executing the applications 108 to adjust or select flow control 64. The control module 66 may provide output signals through the output 82 to the flow control 64 to achieve a selected flow from the ventricles 22. At a selected period, such as during a checkup visit, a user may receive data from the control system 60 and/or transmit signals and data to the control system 60 to alter operation of the valve 30 and/or confirm a selected or optimal operation of the valve 30 of the shunt system 14.

The power source 76 may be provided in an appropriate manner for assisting and providing power to the selected portions of the control system 60, such as the control module 66, the memory 72, and the sensor 68. The power source 76 may include a selected cell battery. The power source 76 may be operable or configured to power operation of the control system 60, transmitting and/or receiving signals from the control system 60, and/or receiving and sending signals from the sensor 44. Accordingly, the power source 76 may be the power source for the shunt system 14 to operate the flow control 64 and/or receive signals regarding operation of the control 64.

The shunt system 14, as discussed above, may be operated by executing the applications 108 with the control system 60 to adjust the flow control 64. As discussed above, the sensors 44 may be implanted in the subject 10, such as positioned within the brain 24. The sensors 44 may be positioned in any appropriate portion on the brain 24 and sense selected brain physiological activity, such as a functional brain activity, electrical brain activity, or the like.

The sensor 44 may be positioned in any appropriate location within the brain 24 that may be monitored with the sensor 44 to assist in operation of the flow control 64 to achieve optimal results. Generally, a selected pressure may be determined to be achieved within the ventricle 22 that allows for the subject 10 to achieve a substantially normal life and observe or have no observable negative side effects from hydrocephalus. Accordingly, the sensor 44 may be configured to sense brain activity within the brain 24 to assist in operating the valve 30 to achieve the selected pressure which may relate to the optimal or desirable outcomes for the subject 10. As noted above, selected or optimal outcomes may include reduced pain, increased cognitive function, etc.

The sensor 44 may sense the brain activity in the brain 24. The sensor 44 may transport or send a sensor signal to the control system 60 based the sensed brain activity. The sensor data in the sensor signal may then be provided to the memory 72, as discussed above, as sensor data 100 and the application 104 may be executed to operate the flow control 64 via or through an output signal of the output module 82.

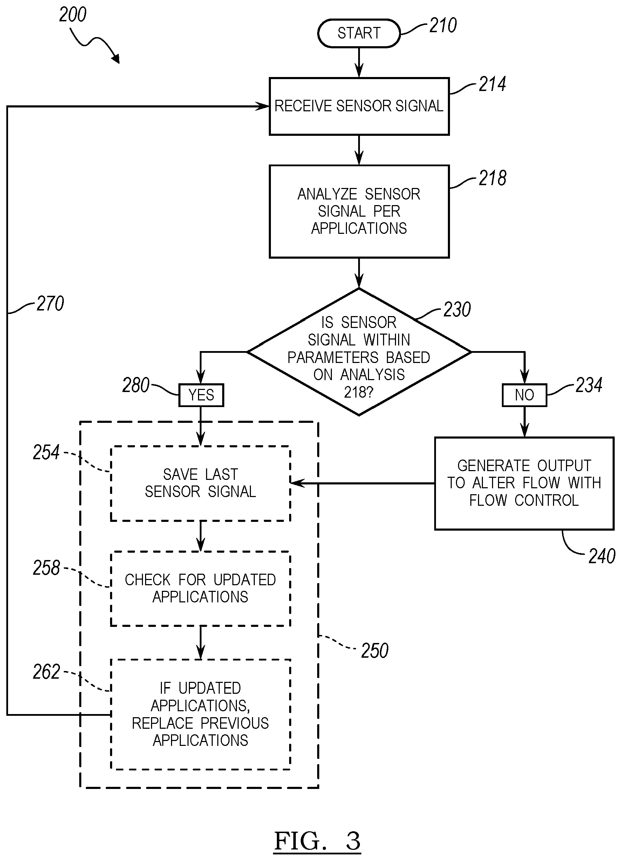

With continuing reference to Fig. 2, and additional reference to Fig. 3, the control system 60 may be operated as illustrated in a flow chart 200. The control module 66 including the data process module 90 may analyze and/or evaluate the senor data 100 to control the flow control 64 according to the method 200. Accordingly, the method 200 may begin in start block 210. Beginning at start block 210 may include various programming, implantation, or the like of the shunt system 14, as discussed further herein. The start block 200 may include an initial or initiation of operation of the shunt system 14 including the valve 30.

The shunt system 14 may be implanted with the sensor 44 and therefore the sensor 44 may generate a signal that is received in block 214. The sensor signal may be generated by the sensor 44 and transmitted to the control system 60, as discussed above. The sensor 44 may generate sensor information that is provided or stored in the memory as sensor data 100 for analysis and/or evaluation and/or control of the flow control 64 by the applications 108. Accordingly the sensor signal may be analyzed per the applications in block 218.

Analysis of the sensor signal per applications in block 218 may include any appropriate analysis and/or calculations. For example, the sensor signal may be filtered to achieve or analyze the sensor signal. Further the sensor signal may be parameterized and/or otherwise evaluated to determine control of the flow control 64 via the applications 108. Accordingly, the applications may include instructions regarding the various parameters, such as limitations or thresholds regarding the signal from the sensor 44. For example, a selected amount, intensity, or the like of brain activity 24 adjacent or sensed by the sensor 44 may be included in the applications 108. For example, threshold parameters may be stored in the memory 72 and included in the applications and/or accessed by the applications 108. IN various embodiments, a lookup table to selected or predetermined values or thresholds may be saved in the memory and/or included or accessed with the applications 108.

Accordingly, a determination of whether the sensor signals within the threshold parameters and/or within selected parameters in block 230 may be made. The determination of whether the signals within the parameters may be a comparison of the most recent sensor signal compared to the lookup table of sensor parameters, such as a lookup table stored in the memory 72. The comparison in block 230 may further and/or alternatively include a calculation of the sensor signal from the sensor 44 based upon a predetermined calculation or formula. Accordingly an individual calculation relative to the subject 10 may be made based upon various input parameters regarding the subject 10.

Based upon the comparison in block 230, a NO path 234 may be followed if the sensor signal from the sensor 44 is not within the selected or determined parameters in block 230. When the NO path 230 is followed, an output signal, also referred to as a control signal, may be generated to alter flow with the flow control may be performed in block 240. The generated control signal in block 240 may be based upon or determined with the applications 108.

The control signal may be output with the output 82 and used to alter the flow control 64. As discussed above, altering the flow control 64 may increase or decrease a flow through the valve 30. As discussed above, adjustment of the valve may include increasing or decreasing a pressure applied to a ball onto a valve seat. Accordingly, the pressure to open the valve 30 may be increased or decrease and, therefore, a related flow may be achieved through the valve 30. Accordingly, if the sensor signal is not within a predetermined parameter the control signal may be generated to alter the flow control in block 240. Altering the flow control in block 240 may assist in achieving a desired or optimal outcome for the subject 10.

After generating the output in block 240, various optional processes may further occur in the optional processes or sub-processes 250. For example, a first optional process may include saving a last sensor signal in block 254. Saving the last sensor signal may include saving or creating the historical data 104, as discussed above. The historical data may be transmitted to the control/program system 120, via the transceiver 78, as discussed above. The historical data 104 may be used to assist in altering applications and/or parameters for operation of the valve 30.

Alternatively or further, a check for updated applications may be made in block 258. The updated applications may include updated or changed parameters that may be included or provided to the analysis block 218. For example, an updated or new sensor signal parameters may be included, a degree of altering the flow control may be updated, or other appropriate changes may be made in the applications. The control method 200 may include a check for updated applications in block 258. If the applications are updated they will replace the original or previous applications in block 262. Accordingly, the optional process or sub-processes 250 may be used to update the control system 60 based upon operation of the control system 60 and/or the valve 30, and/or the shunt system 14. Further, the subject 10 may change anatomical or physiological features or parameters and the control system 60 may be updated by updating the applications, as discussed above.

The method 200 may then loop with a loop or continuous monitoring path 270 to receive a sensor signal in block 214. The control system 60 may operate according to the method 200 as a continuous loop during a life of the operation of the shunt system 14. The shunt system 14, therefore, may include a continuous feedback loop, which may be a closed feedback loop, to alter the flow control 64 based upon the received sensor signal in block 214. The method 200 may allow for operation of the shunt system 14 according to selected applications to achieve selected outcomes for the subject 10, such as desired or optimal outcomes.

If the sensor signal is within selected parameters in block 230, a YES path 280 may be followed. The YES path 280 may directly go to a received sensor signal in block 214 and/or may go to the optional processes in block 250. The YES path 280 may also include, if selected, a no change control signal. Thus, a control signal may be generated for both the NO path 234 and the YES path 280. The YES path 280, therefore, may also be used to ensure a continuous loop of a selected operation of the shunt system 14. The YES path 280 also allows the method 200 to operate in a substantially continuous loop. Therefore, the method 200, which may be an operation of the control system 60 of the shunt 14, may be operated in a substantially continuous loop to ensure that the sensor signal 44 remains within selected parameters by altering the flow control 64 in block 240.

It is further understood that the control system 60 may provide an output, such as the transceiver 78, to provide warnings and/or updates regarding operation of the shunt system 14. For example, if the determination in block 230 follows the NO path 234 a selected number of times in sequence, or within a selected period of time, or the like, an output signal may be provided to provide a warning to the subject 10 and/or caregiver. Accordingly, the control system 60 may provide output for ensuing operation of the shunt system 14 within selected parameters and/or additional or updated care of the subject 10.

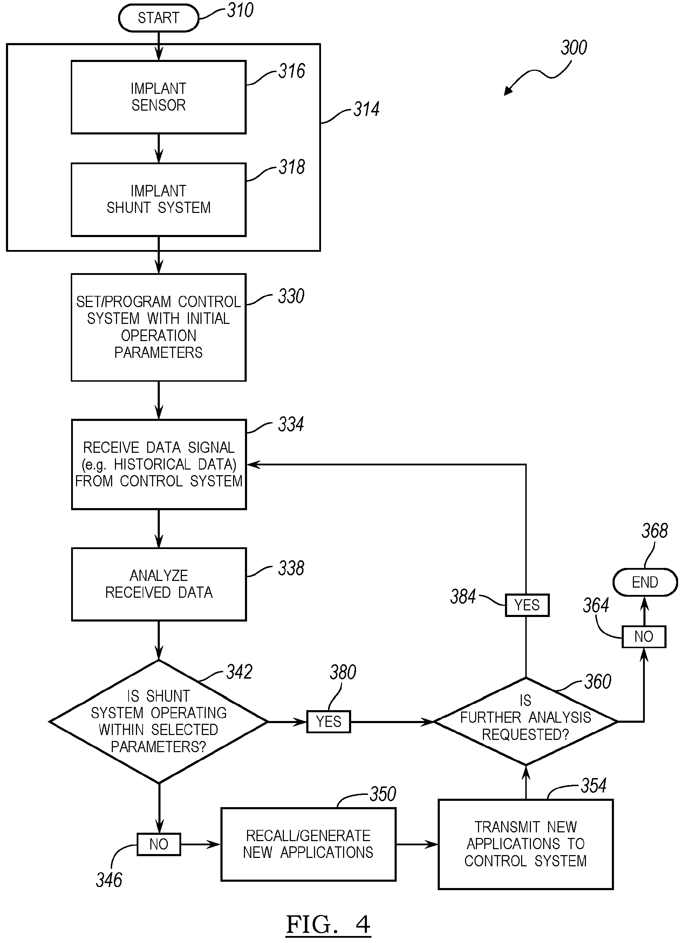

With continuing reference to Figs. 2 and 3, and additional reference to Fig. 4, a process or method 300 for operation or use of the shunt system 14 is illustrated. The process 300 may be considered to be an operation of the shunt system 14, such as by a surgeon and/or clinician in addition to and/or alternatively to operation of the control system 60. The method 200 may be understood to be an operation of the shunt system 14, such as internally within the subject 10, which may be augmented by input or augmentation of the operation of the control system. The method 300, however, may include various operation and alteration of the shunt system 14.

Generally the method 300 begins at start block 310. The start block 310 may include any appropriate steps, such as analysis of the subject 10, diagnosis of the subject 10, selection of optimal treatment conditions (e.g. subject life outcomes, physical achievements, CSF pressure, etc.). In various embodiments, such as after a diagnosis of hydrocephalus of the subject 10, a sub-process or initial process of implantation in block 314 may be selected. The implantation in block 314 may be selected, however, but may not be necessary for the method 300. The implantation sub-process 314 may occur separately and/or alternatively to the remainder of the method 300. Generally an implantation sub-process 314 a sensor is implanted in block 316. Further a shunt system is implanted in block 318. The sensor implanted in block 316 may include the brain function or activity sensor 44, as discussed above. The sensor 44 may be implanted in block 316 separate from the shunt system 14 and/or incorporated into various features, such as the inlet catheter 18, or other appropriate configuration. In various embodiments, for example, the inlet catheter 18 may include the sensor 44 as a feature or portion therewith (e.g. an electrode on a selected portion of the inlet catheter 18) and, therefore, may be implanted substantially simultaneously with a shunt system. The shunt system implanted in block 318 may be implanted in a selected manner, including either or both of the valve 30 and/or the pump 34. Further, the implantation of the shunt system 14 may include a selection of an inlet location for the inlet catheter 18 and an outlet location for the outlet catheter 36.

Regardless of the implantation procedure 314, the control system 60 may initially be set or programmed with initial operation parameters in block 330. The initial parameters may include selected brain activity for a given flow control position, limits to the flow control positioning, duty cycle for the control system (e.g. monitoring frequency of the sensor 44), and other appropriate parameters. The initial parameters may be set by a clinician, such as the surgeon performing the implantation of the shunt system 14, a clinician following the progress of the subject 10, or any appropriate individual. The initial setup and program may be performed with the control/program system 120, as discussed above.

In other words, the initial parameters in block 330 may be initial desired parameters programmed into the system. These initial parameters may be initially set up by the clinician (e.g. physician) based on patient symptomology. Specific initial parameters and/or their values may vary from one patient to the next. The physician and/or patient alone and/or together may decide the optimal initial settings. The optimal initial settings may be or include some initial "tuning" based on patient feedback and physician analysis and experience. After the initial setup in block 330, the system controls the flow control (e.g. the valve 30 and/or pump 34) settings to maintain the sensor signal from the monitored parameters (e.g. brain activity with the sensor 44) in a range that minimizes patient symptoms. Thus, the control system 60 may operate, as discussed above, to achieve the optimal flow control based on the feedback. Addition, feedback may include patient feedback (e.g. reporting of undesired outcomes (e.g. pain, dizziness, etc.) and/or desired outcomes (e.g. lack of pain, etc.)).

During operation of the control system 60 of the shunt system 14, as discussed above, selected data, such as historical data 104 and/or feedback data 110 may be collected. At a selected time, such as at a checkup of the subject 10, a receiving of a data signal from the control system 60 may be made into a selected system, such as the control/program system 120 in block 334. The receiving of a signal in block 334 may include a download or transmittal of selected data, such as the historical data 104 from the control system 60. As discussed above the transceiver 76 may be set to transmit the historical data a selected time and/or after receiving a signal, such as a signal from the control/program system 120. Regardless, receipt of data from the control system 60 may be made.

An analysis of the received data may be made in block 338. The analysis of the data in block 338 may be whether the shunt is operating within selected parameters, for which a determination may be made in block 342. Operation of the shunt system within selected parameters may include a determination that the flow control 64 is adjusted at a selected rate or over a selected period, the control system 60 includes only a selected number of NO determinations in block 234 over a selected period of time, or other appropriate parameters. It is understood, the operational parameters of the shunt system 14 may be selected and/or may be based upon the selected and individual subject 10.

If it is determined that the shunt operation system is not within operational parameters, a NO path 346 may be followed to recall or generate new applications in block 350. As discussed above, applications may include instructions on operation of the shunt system 14, such as how and when to change or alter the flow control 64. The new applications may be generated based upon a received data, generally known or determined parameters for operation of the shunt system 14, or other appropriate inputs. Accordingly, new applications may be generated or recalled based upon the individual data received, a plurality of data's received from a plurality of a subject, or other appropriate determinations.

The new applications may be transmitted to the control system, such as the memory 72, in block 354. The transmission of the new applications to the control system 60 in block 354 may again be performed via the transceiver 78 and may be stored for execution by the control module 66, as discussed above.

A determination of whether further analysis is requested or required in block 360 may then be made. The further analysis may include a short follow up, a confirmation of receipt of the new applications, or other appropriate further analysis. If no further analysis is requested a NO path 364 may be followed to end the process 300 in block 368. Ending the process in block 368 may include any appropriate additional procedures, such as restarting the control system 60, recharging the power source 76, or other appropriate and further actions. Accordingly, the end block 368 is understood to include or selectively include additional features or procedures as selected or required.

Returning to the determination block 342, if the shunt system is operating within selected parameters a YES path 380 may be followed. The YES path 380 may also go to a determination of whether further analysis is required in block 360. Accordingly, the NO path 364 may be followed to the end block 360.

At the determination of whether further analysis is required in block 360, an alternative and/or additional path may also include a YES path 384. The YES path 384 may return to receive additional data from the control system in block 334. The data may be data collected after execution of the new applications from block 350, if the NO path 346 is followed, or further collection of data if the YES path 380 is followed. The selection of following the YES path to collect additional data in block 334, however, may be selected according to any appropriate procedure, and may be based upon the clinician's selection.

In light of the above, therefore, the shunt system 14 may be implanted into the subject 10. The shunt system 14 may include a selected feedback loop, such as based upon the sensor 44 and the signal therefrom, to collect data regarding the brain 24 and alter and/or maintain operation of the valve 30. Accordingly, the shunt system 14 may include a feedback loop to achieve or attempt to achieve optimal or selected outcomes for the subject 10. The control system 10 may include a control system 60, which may be a selected processor module, to execute selected instructions to achieve appropriate outcomes and/or include control of the flow control 64. Further, the shunt system 14 may be operated and selected by a user or clinician to assist in achieving the outcomes.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The foregoing description of the embodiments has been provided for purposes of illustration and description. It is not intended to be exhaustive or to limit the invention. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the invention, and all such modifications are intended to be included within the scope of the invention.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, graphic processing units (GPUs), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

## Claims

1. A system for controlling a hydrocephalus shunt system (14), comprising:
a sensor (44) configured to sense a physiological activity of a subject (10) and generate a sensor signal related thereto;
a control system (60) comprising:
a control module (66) configured to execute instructions;
a memory module (72) configured to store the instructions;
an input connection configured to receive the sensor signal from the sensor (44);
wherein the control module (66) is operable to evaluate and/or analyze the sensor signal to generate a control signal to operate a flow control (30; 34),
wherein the sensor (44) is configured to sense a brain activity of the subject (10).

2. The system of Claim 1, further comprising:
the flow control (30; 34), wherein the flow control (30; 34) is configured to control a flow of a material through the hydrocephalus shunt system (14), wherein, optionally:
the flow control (30; 34) is a pump (34), or
the flow control (30; 34) is a valve (30), wherein, further optionally, the valve (30) includes a valve portion configured to be adjusted to change an opening pressure of the valve (30) based on the control signal of the control module (66).

3. The system of any one of Claims 1 or 2, further comprising:
a catheter (16) configured to be positioned in a ventricle of a brain (24) of the subject (10);
wherein the sensor (44) is formed integrally with the catheter (16).

4. The system of any one of Claims 1 to 3, wherein the control module (66) configured to execute instructions includes comparing the sensor signal to look up table of parameters stored in the memory module (72).

5. The system of any one of Claims 1 to 4, wherein the control module (66) configured to execute instructions includes:
determining whether the sensor signal is within a selected threshold;
when the sensor signal is outside of the selected threshold, generating the control signal to change operation of the flow control (30; 34).

6. The system of any one of Claims 1 to 5, wherein the control module (66) configured to execute instructions includes:
storing historical data regarding the sensor signal over time.

7. The system of any one of Claims 1 to 6, wherein the control system (60) further comprises a transceiver (78) configured to at least one of transmit a signal from the control system (60) or receive a signal.

8. The system of any one of Claims 1 to 7, further comprising:
a programmer (120) separate from the control system (60) configured to at least one of transmit a signal to the control system (60) or receive a signal from the control system (60), wherein, optionally, the programmer (120) is configured to transmit a new application including instructions on operation of the system to the control system (60) to evaluate and/or analyze the sensor signal to generate the control signal to operate the flow control (30; 34).

9. The system of any one of Claims 1 to 8, wherein:
the control module (66) configured to execute the instructions to:
receive the sensor signal from the sensor (44);
recall instructions from the memory module (72); and
generate the control signal to operate the flow control (30; 34) based on the evaluation and/or analyze the sensor signal, wherein, optionally,
the system further comprises:
configuring the sensor (44) for implantation in a brain (24) of the subject (10).

10. The system of Claim 9, wherein the control module (66) is further configured to:
transmit the control signal to control the flow control (30; 34);
wherein the flow control (30; 34) is a valve (30).

11. The system of Claim 10, wherein the valve (30) is configured as the flow control (30; 34).

12. The system of Claim 9, wherein the control module (66) is further configured to:
transmit the control signal to control the flow control (30; 34);
wherein the flow control (30; 34) is a pump (34).

13. The system of Claim 12, further comprising:
a pump (34) configured as the flow control (30; 34).

14. The system of any one of Claims 9 to 13, wherein the control module (66) is further configured to:
generate a new application including instructions on operation of the system;
transmitting the new application to the memory module (72); and
evaluate and/or analyze the sensor signal to generate a control signal to operate the flow control (30; 34).

15. The system of any one of Claims 9 to 14, wherein the control module (66) is further configured to:
generate a warning signal regarding at least one of operation of the control module (66), the received sensor signal, the generated control signal, or combinations thereof.

## Patentansprüche

1. System zum Steuern eines Hydrocephalus-Shunt-Systems (14), umfassend:
einen Sensor (44), der konfiguriert ist, um eine physiologische Aktivität eines Subjekts (10) zu erfassen und ein Sensorsignal, das sich darauf bezieht, zu generieren;
Steuerungssystem (60), umfassend:
ein Steuerungsmodul (66), das konfiguriert ist, um Anweisungen auszuführen;
ein Speichermodul (72), das konfiguriert ist, um die Anweisungen zu speichern;
einen Eingangsanschluss, der konfiguriert ist, um das Sensorsignal von dem Sensor (44) zu empfangen;
wobei das Steuerungsmodul (66) betriebsfähig ist, um das Sensorsignal auszuwerten und/oder zu analysieren, um ein Steuerungssignal zu generieren, um eine Flusssteuerung (30; 34) zu betreiben,
wobei der Sensor (44) konfiguriert ist, um eine Gehirnaktivität des Subjekts (10) zu erfassen.

2. System nach Anspruch 1, ferner umfassend:
die Flusssteuerung (30; 34), wobei die Flusssteuerung (30; 34) konfiguriert ist, um einen Fluss eines Materials durch das Hydrocephalus-Shunt-System (14) zu steuern, wobei optional:
die Flusssteuerung (30; 34) eine Pumpe (34) ist oder
die Flusssteuerung (30; 34) ein Ventil (30) ist, wobei ferner optional das Ventil (30) einen Ventilabschnitt einschließt, der konfiguriert ist, um angepasst zu werden, um einen Öffnungsdruck des Ventils (30) basierend auf dem Steuerungssignal des Steuerungsmoduls (66) zu ändern.

3. System nach einem der Ansprüche 1 oder 2, ferner umfassend:
einen Katheter (16), der konfiguriert ist, um in einem Ventrikel eines Gehirns (24) des Subjekts (10) positioniert zu werden;
wobei der Sensor (44) mit dem Katheter (16) einstückig ausgebildet ist.

4. System nach einem der Ansprüche 1 bis 3, wobei das Steuerungsmodul (66), das konfiguriert ist, um Anweisungen auszuführen, ein Vergleichen des Sensorsignals mit einer Nachschlagetabelle von Parametern, die in dem Speichermodul (72) gespeichert sind, einschließt.

5. System nach einem der Ansprüche 1 bis 4, wobei das Steuerungsmoduls (66), das konfiguriert ist, um Anweisungen auszuführen, einschließt:
Bestimmen, ob das Sensorsignal innerhalb eines ausgewählten Schwellenwerts liegt;
wenn das Sensorsignal außerhalb des ausgewählten Schwellenwerts liegt, Generieren des Steuerungssignals, um den Betrieb der Flusssteuerung (30; 34) zu ändern.

6. System nach einem der Ansprüche 1 bis 5, wobei das Steuerungsmoduls (66), das konfiguriert ist, um Anweisungen auszuführen, einschließt:
Speichern von historischen Daten bezüglich des Sensorsignals im Laufe der Zeit.

7. System nach einem der Ansprüche 1 bis 6, wobei das Steuerungssystem (60) ferner einen Transceiver (78) umfasst, der konfiguriert ist, um mindestens eines von einem Signal von dem Steuerungssystem (60) zu übertragen oder ein Signal zu empfangen.

8. System nach einem der Ansprüche 1 bis 7, ferner umfassend:
einen Programmierer (120), der von dem Steuerungssystem (60) getrennt ist, der konfiguriert ist, um mindestens eines von einem Signal an das Steuersystem (60) zu übertragen oder ein Signal von dem Steuerungssystem (60) zu empfangen, wobei optional der Programmierer (120) konfiguriert ist, um eine neue Anwendung, einschließlich Anweisungen zum Betrieb des Systems, an das Steuersystem (60) zu übertragen, um das Sensorsignal auszuwerten und/oder zu analysieren, um das Steuerungssignal zu generieren, um die Flusssteuerung (30; 34) zu betreiben.

9. System nach einem der Ansprüche 1 bis 8, wobei:
das Steuerungsmodul (66) konfiguriert ist, um Anweisungen auszuführen zum:
Empfangen des Sensorsignals von dem Sensor (44);
Abrufen von Anweisungen aus dem Speichermodul (72); und
Generieren des Steuerungssignals, um die Flusssteuerung (30; 34) basierend auf der Auswertung und/oder Analyse des Sensorsignals zu betreiben, wobei optional
das System ferner umfasst:
Konfigurieren des Sensors (44) für eine Implantation in das Gehirn (24) des Subjekts (10).

10. System nach Anspruch 9, wobei das Steuerungsmodul (66) ferner konfiguriert ist zum:
Übertragen des Steuerungssignals, um die Flusssteuerung (30; 34) zu steuern;
wobei die Flusssteuerung (30; 34) ein Ventil (30) ist.

11. System nach Anspruch 10, wobei das Ventil (30) als Flusssteuerung (30; 34) konfiguriert ist.

12. System nach Anspruch 9, wobei das Steuerungsmodul (66) ferner konfiguriert ist zum:
Übertragen des Steuerungssignals, um die Flusssteuerung (30; 34) zu steuern;
wobei die Flusssteuerung (30; 34) eine Pumpe (34) ist.

13. System nach Anspruch 12, ferner umfassend:
eine Pumpe (34), die als Flusssteuerung (30; 34) konfiguriert ist.

14. System nach einem der Ansprüche 9 bis 13, wobei das Steuerungsmodul (66) ferner konfiguriert ist zum:
Generieren einer neuen Anwendung einschließlich Anweisungen für den Betrieb des Systems;
Übertragen der neuen Anwendung an das Speichermodul (72); und
Auswerten und/oder Analysieren des Sensorsignals, um ein Steuerungssignal zu generieren, um die Flusssteuerung (30; 34) zu betreiben.

15. System nach einem der Ansprüche 9 bis 14, wobei das Steuerungsmodul (66) ferner konfiguriert ist zum:
Generieren eines Warnsignals bezüglich mindestens eines von einem Betrieb des Steuerungsmoduls (66), des empfangenen Sensorsignals, des generierten Steuerungssignals oder einer Kombination davon.

## Revendications

1. Système permettant de commander un système de dérivation d'hydrocéphalie (14), comprenant :
un capteur (44) configuré pour détecter une activité physiologique d'un sujet (10) et générer un signal de capteur en rapport avec celle-ci ;
un système de commande (60) comprenant :
un module de commande (66) configuré pour exécuter des instructions ;
un module de mémoire (72) configuré pour stocker les instructions ;
une connexion d'entrée configurée pour recevoir le signal de capteur à partir du capteur (44) ;
dans lequel le module de commande (66) permet d'évaluer et/ou d'analyser le signal de capteur afin de générer un signal de commande pour faire fonctionner une commande de débit (30 ; 34),
dans lequel le capteur (44) est configuré pour détecter une activité cérébrale du sujet (10).

2. Système selon la revendication 1, comprenant en outre :
la commande de débit (30 ; 34), dans lequel la commande de débit (30 ; 34) est configurée pour commander le flux d'un matériau à travers le système de dérivation d'hydrocéphalie (14), dans lequel, éventuellement :
la commande de débit (30 ; 34) est une pompe (34), ou
la commande de débit (30 ; 34) est une vanne (30), dans lequel, en outre éventuellement, la vanne (30) comporte une partie de vanne conçue pour être ajustée afin de modifier une pression d'ouverture de la vanne (30) en fonction du signal de commande du module de commande (66).

3. Système selon l'une quelconque des revendications 1 ou 2, comprenant en outre :
un cathéter (16) conçu pour être positionné dans un ventricule d'un cerveau (24) du sujet (10) ;
dans lequel le capteur (44) est formé d'un seul tenant avec le cathéter (16).

4. Système selon l'une quelconque des revendications 1 à 3, dans lequel le module de commande (66) configuré pour exécuter des instructions comporte la comparaison du signal de capteur à la table de consultation des paramètres stockés dans le module de mémoire (72).

5. Système selon l'une quelconque des revendications 1 à 4, dans lequel le module de commande (66) configuré pour exécuter des instructions comporte :
la détermination si le signal de capteur se situe dans un seuil sélectionné ;
lorsque le signal de capteur est en dehors du seuil sélectionné, la génération du signal de commande pour modifier le fonctionnement de la commande de débit (30 ; 34).

6. Système selon l'une quelconque des revendications 1 à 5, dans lequel le module de commande (66) configuré pour exécuter des instructions comporte :
le stockage des données historiques concernant le signal de capteur au fil du temps.

7. Système selon l'une quelconque des revendications 1 à 6, dans lequel le système de commande (60) comprend en outre un émetteur-récepteur (78) configuré pour au moins l'une parmi les opérations suivantes : transmettre un signal du système de commande (60) ou recevoir un signal.

8. Système selon l'une quelconque des revendications 1 à 7, comprenant en outre :
un programmateur (120) séparé du système de commande (60) configuré pour au moins l'une parmi les opérations suivantes : transmettre un signal au système de commande (60) ou recevoir un signal à partir du système de commande (60), dans lequel, éventuellement, le programmateur (120) est configuré pour transmettre une nouvelle demande comportant des instructions sur le fonctionnement du système au système de commande (60) pour évaluer et/ou analyser le signal de capteur afin de générer le signal de commande pour faire fonctionner la commande de débit (30 ; 34).

9. Système selon l'une quelconque des revendications 1 à 8, dans lequel :
le module de commande (66) est configuré pour exécuter les instructions pour :
recevoir le signal de capteur à partir du capteur (44) ;
rappeler les instructions à partir du module de mémoire (72) ; et
générer le signal de commande pour faire fonctionner la commande de débit (30 ; 34) en fonction de l'évaluation et/ou de l'analyse du signal de capteur, dans lequel, éventuellement,
le système comprend en outre :
la configuration du capteur (44) pour l'implanter dans un cerveau (24) du sujet (10).

10. Système selon la revendication 9, dans lequel le module de commande (66) est en outre configuré pour :
transmettre le signal de commande pour commander la commande de débit (30 ; 34) ;
dans lequel la commande de débit (30 ; 34) est une vanne (30).

11. Système selon la revendication 10, dans lequel la vanne (30) est configurée en tant que la commande de débit (30 ; 34).

12. Système selon la revendication 9, dans lequel le module de commande (66) est en outre configuré pour :
transmettre le signal de commande pour commander la commande de débit (30 ; 34) ;
dans lequel la commande de débit (30 ; 34) est une pompe (34).

13. Système selon la revendication 12, comprenant en outre :
une pompe (34) configurée en tant que la commande de débit (30 ; 34).

14. Système selon l'une quelconque des revendications 9 à 13, dans lequel le module de commande (66) est en outre configuré pour :
générer une nouvelle demande comportant des instructions sur le fonctionnement du système ;
transmettre la nouvelle demande au module de mémoire (72) ; et
évaluer et/ou analyser le signal de capteur afin de générer un signal de commande pour faire fonctionner la commande de débit (30 ; 34).

15. Système selon l'une quelconque des revendications 9 à 14, dans lequel le module de commande (66) est en outre configuré pour :
générer un signal d'avertissement concernant au moins l'un parmi le fonctionnement du module de commande (66), le signal de capteur reçu, le signal de commande généré, ou des combinaisons de ceux-ci.
